# EUROPÄISCHE PATENTSCHRIFT

(11) **EP 1 627 882 B1**
(45) Veröffentlichungstag und Bekanntmachung des Hinweises auf die Patenterteilung: **22.11.2006**
(21) Anmeldenummer: 05009987.8
(22) Anmeldetag: 07.05.2005
(51) Int. Cl.: C07H 15/04, C07H 15/06, C07H 15/10

(54) **Verfahren zur Herstellung von Alkylglycosiden**
Process for the preparation of alkypolyglycosides
Procédé de préparation d'alkylpolyglycosides

(30) Priorität: 22.05.2004 DE 102004025195
(43) Veröffentlichungstag der Anmeldung: 22.02.2006
(73) Patentinhaber: Goldschmidt GmbH, 45127 Essen (DE)
(72) Erfinder: Pascaly, Matthias, Dr., 48163 Münster (DE); Grüning, Burghard, Dr., 45134 Essen (DE); Maass, Dietrich, Dr., 48341 Altenberge (DE); Weitemeyer, Christian, Dr., 45257 Essen (DE)

(56) Entgegenhaltungen:
- EP-A- 0 729 970
- EP-A- 0 970 097
- WO-A-93/22324
- US-A- 3 346 558
- US-A- 4 223 129
- US-A- 5 554 740

## Beschreibung

Glykoside ist die Sammelbezeichnung für eine umfangreiche Gruppe von Pflanzenstoffen und synthetischen Verbindungen, die durch Kochen mit Wasser oder verdünnten Säuren oder unter der Einwirkung von Glykosidasen in ein oder mehrere Kohlenhydrate (Mono- oder Oligosaccharide) und andere Verbindungen gespalten werden. Die Zucker sind durch ein Sauerstoff-Atom in einer glykosidischen Bindung (gen. Ether-Bindung) an ein Halbacetal-C-Atom zum Vollacetal gebunden. (Römpp Lexikon Chemie - Version 2.0, Stuttgart/New York: Georg Thieme Verlag 1999).

Das Monomer der Glycoside ist meist ein Monosaccharid, wie z. B. Glucose (am häufigsten) oder Galactose, Mannose, Fructose u.a. Hexosen oder die Pentosen Arabinose, Xylose, Ribose; daneben kommen auch Zucker vor, die ausschließlich in Glycosiden gefunden werden, so z.B. Digitalose, Cymarose usw. Ist der Kohlenhydrat-Rest eines Glycosid eine Glucose, so nennt man das Derivat ein Glucosid; analog sind die Fructoside Glycoside mit Fructose, Galactoside Glycoside mit Galactose als Zucker-Komponenten. (Römpp Lexikon Chemie - Version 2.0, Stuttgart/New York: Georg Thieme Verlag 1999).

Methylglycoside sind Ausgangsstoffe für die Synthese von Alkylglycosiden. Das sind Vollacetale, die aus Zucker-Halbacetalen hergestellt werden. Analog werden aus den nicht reduzierenden Zuckern entsprechende Ketale gebildet (Lit.: A. T. J. W. de Goede, F. van Rantwijk, H. Van Bekkum, Starch/Stärke 47 (1995) 233 - 237). Es handelt sich dabei um eine überaus wichtige Stoffklasse für die kosmetische Industrie. Einsatz finden die bei weitem wichtigsten Verbindungen (evtl. nach weiteren Modifikationen), insbesondere als nicht-ionische Tenside, mit guten Schaumeigenschaften, als Emulgatoren oder auch als Verdicker. Der Vorteil dieser Verbindungen ist deren geringes Schleimhaut-Reizpotential sowie deren biologische Abbaubarkeit.

Die klassische Synthese von Alkylglucosiden erfolgt durch Umsetzung von Zuckern oder Kohlenhydraten mit Alkoholen in Anwesenheit eines sauren Katalysators (Fischer Glycosidierung; Lit.: A. F. Bochkov, G. E. Zaikov: The Chemistry of the O-Glycosidic Bond, Pergamon Press, Oxford, 1979, 210 pp.). Um eine Reaktion der miteinander nicht mischbaren Edukte zu ermöglichen wird das Gemisch lange Zeit bei hoher Temperatur gehalten und das entstehende Wasser abgezogen. Bereits die einfache Hydrolyse eines Kohlenhydrates erfordert relativ drastische Bedingungen durch Verwendung von 1M Schwefelsäure und 100 °C für mehrere Stunden (gilt für Hexose-enthaltende Polysaccharide; Lit.: Frieder W. Lichtenthaler, in: Ullmann's Enzyclopedia of Industrial Chemistry, "Carbohydrates"). Dabei kann eine teilweise Zersetzung der Zucker jedoch nicht verhindert werden. Generell hängt die Produktzusammensetzung stark von dem gewählten Katalysator ab. In einer zweistufigen Synthese kann zunächst ein Glycosid mit kurzer Alkylkette hergestellt werden, welche anschließend durch Umacetalisierung in Anwesenheit einer Säure durch einen anderen Alkylrest ausgetauscht wird.

Typische Katalysatoren für die Alkylierung von Glycosiden sind Schwefelsäure und p-Toluolsulfonsäure wie in US 3 772 269 und US 3 375 243 beschrieben. Solche starken Säuren verursachen intensive Färbung des Produktes, was eine Aufarbeitung des Produktes erforderlich macht. Ferner ist die Verwendung von Mischsalzen aus einer starken organischen Säure und einer schwachen organischen Base bekannt (US 5 432 269), jedoch sind auch die derart hergestellten Produkte stark gefärbt und können freie organische Basen enthalten. Auch Salze von mehrbasigen Carbonsäuren (US 4 898 934) sowie Hydroxycarbonsäuren (US 4 465 828) werden als Katalysatoren verwendet. Beim Einengen des Reaktionsgemisches wird jedoch unweigerlich ein gefärbtes Produkt erhalten durch die thermische Belastung in Gegenwart einer sich aufkonzentrierenden Säure. Daher muß in allen Fällen ein Schritt zur Entfärbung des Produktes (z.B. mit H₂O₂) angeschlossen werden.

Das einfachste und billigste Glycosid ist Methylglucosid (siehe Formel II in Schema 1), dessen Herstellung schon sehr lange bekannt ist. Siehe Römpp Chemie Lexikon, Stichpunkt: α-Methylglucosid: Herstellung durch Einwirkung von Methanol auf Glucose in Gegenwart von HCl oder einem Kationenaustauscher. Daneben entsteht noch β-Methylglucosid. Schema 1:

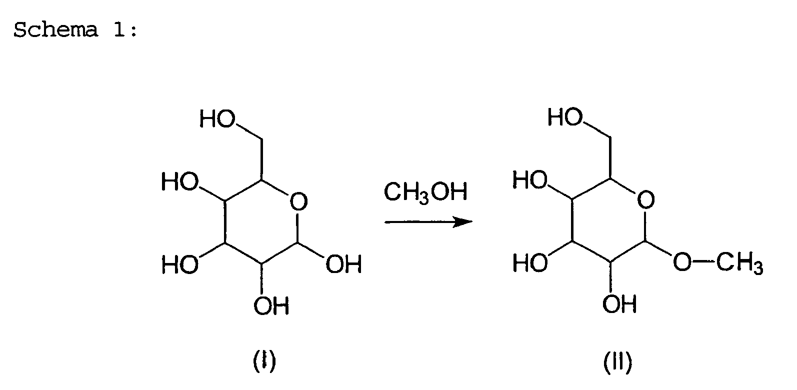

Diese Synthese ist auch in zahlreichen Patentschriften beschrieben, beispielsweise in der US 2 276 621 (Veröffentlichungsdatum 17.03.1942). Die Reaktion wird in Methanol als alkoholische Komponente und gleichzeitig Lösungsmittel in Anwesenheit eines Kohlenhydrates wie beispielsweise Stärke durchgeführt. Als Katalysatoren werden anorganische Säuren wie z.B. Schwefelsäure verwendet. Das Kohlenhydrat hydrolysiert zu Glucose und in Anwesenheit des Katalysators wird Methylglucosid erhalten.

In der JP-A-06-092984 ist die Verwendung eines immobilisierten Katalysatorsystems auf Basis eines Kationenaustauscherharzes zur Herstellung von Methylglucosid beschrieben, was eine Darstellung nur geringfügig gefärbter Produkte ermöglichen soll. Analog dazu ist die Verwendung von Amberlyst 15 in der DE-A-3 611 035 beschrieben.

In der FR-1 114 382 ist die Umsetzung von Kartoffelstärke mit Methanol in Gegenwart von HCl beschrieben. Eine weitere Umsetzung erfordert die Zugabe verdünnter Schwefelsäure sowie anschließende Entfärbung.

In der US-A-4 223 129 ist die Herstellung von Alkylpolyglycosiden aus Kartoffelstärke mit Alkohol in Gegenwart eines Katalysators beschrieben, wobei zuerst Kartoffelstärke und Alkohol als "slurry" verarbeitet werden, bei erhöhtem Temperatur (100-400°C) vorzugsweise 160-180 °C im Falle von Methanol, und unter Druck, so dass die Reaktionszeit verkürzt wird und eine Repolymerizierung der Polyglycosiden vermieden wird und eine zusätzliche Bleichaufarbeitung nicht erforderlich ist.

Eine Methode zur Darstellung von Alkylglycosiden direkt aus Oligo- oder Polyglycosiden ist nach dem Stand der Technik nur in einem langwierigen Prozess durch hydrolytische Spaltung (enzymatisch z.B. mit Glycosidasen oder chemisch durch Säurekatalyse) in Anwesenheit eines Katalysators in Alkoholen möglich. Dabei fallen bei Verwendung der o.g. üblichen Methoden eine Vielzahl von Nebenprodukten an sowie zum Teil intensiv gefärbte Produkte (hauptsächlich unreagierte Zucker), wodurch eine weitere Aufarbeitung des Produktes erforderlich wird.

Die Herstellung von Alkylpolyglycosiden ist z.B. in der EP-B-0 970 097 beschrieben. Für die Bildung des Vollacetals wird ein Monosaccharid mit einem Überschuss Alkohol in Gegenwart eines Gemisches aus Schwefelsäure und Natriumhydroxid oder Natriumcarbonat als Katalysator bei hoher Temperatur und Normaldruck umgesetzt. Obwohl normalerweise eine Neutralisation des Reaktionsgemisches durch geeignete Wahl des Katalysatorgemisches nicht nötig sein sollte, kann auch in diesem Fall eine Zugabe von Basen notwendig sein. Als Alkohole können sämtliche ein oder mehrwertige primäre oder sekundäre Alkohole eingesetzt werden. Ferner ist die enzymatische Glycosidierung bekannt (JP-A-9087294, EP-A-725 144, JP-A-806 769 0, JP-A-708 799 2).

Auch die Umsetzung unterschiedlichster Zucker z.B. Maltose - auch polymerer Natur - ist bekannt. Die Säure-katalysierte Umsetzung von Malto-Oligosacchariden mit Alkoholen oder Thiolen ist in der US-A-2002/099 185 beschrieben.

Bei allen diesen Darstellungs-Verfahren handelt es sich jedoch um zeitaufwendige Batch-Synthesen der entsprechenden Alkylglycoside, bei denen auf Grund der harschen Bedingungen, denen die Edukte über lange Zeit ausgetzt sind, zahlreiche Nebenprodukte, die eine intensive Färbung des Produktes hervorrufen, erhalten werden können.

Es bestand daher also weiterhin Bedarf nach einer Methode zur einfachen und kostengünstigen Synthese von Alkylglycosiden, bei der diese Produkte in hohen Raum-Zeit-Ausbeuten kontinuierlich oder diskontinuierlich durch direkte Umsetzung von Zuckern, Oligo- oder Polyglycosiden, möglichst ohne Zusätze von Katalysatoren oder Aktivatoren, so hergestellt werden können, dass eine weitere Aufarbeitung, beispielsweise mit Bleichmitteln, nicht erforderlich ist.

Eine Aufgabe der Erfindung war es, ein solches Verfahren zur Verfügung zu stellen.

Es wurde nun überraschenderweise gefunden, dass sich natürliche und nicht natürliche Glycoside sowie Oligo- und Polyglycoside in Alkoholen bei hohen Drücken und hohen Temperaturen, dabei müssen beide Parameter mindestens die kritischen Werte des Alkohols erreichen, durch Reaktion der Carbonylgruppe mit dem Alkohol reagieren, was letztlich einer einfachen Reaktion des Halbacetals zu einem Vollacetal entspricht. Ferner wurde gefunden, dass sich Polyglycoside unter diesen Bedingungen in ihre Grundbausteine, die Glycoside, spalten lassen, unter Bildung voll acetalisierter Monomerer (vgl. Schema 1). Säuregruppen enthaltende Glycoside wie beispielsweise Pectine werden unter den gewählten Bedingungen auch verestert.

Ein Gegenstand der Erfindung ist ein Verfahren zur Herstellung von Alkylpolyglycosiden aus monomeren Glycosiden, Oligo- oder Polyglycosiden und Alkoholen, nach dem allgemeinen Schema

(Glyc-O)_{z}H + R¹-OH → (Glyc-O)_{z},R¹

worin bedeuten
- z: ≥ 1,
- z': ≤ z, vorzugsweise 1 bis 10,
- (Glyc-O)-: ein Glycosidrest,
- R¹: ein gegebenenfalls Mehrfachbindungen und/oder Heteroatome enthaltender Kohlenwasserstoffrest, welcher dadurch gekennzeichnet ist, dass die Reaktion unter überkritischen Bedingungen, bezogen auf den Alkohol, durchgeführt wird.

Weitere Gegenstände der Erfindung werden durch die Ansprüche gekennzeichnet.

Bei der erfindungsgemäßen Umsetzung von monomeren Glycosiden sowie Oligo- und Polyglycosiden werden durch Reaktion in überkritischen Alkoholen die Vollacetale gemäß dem allgemeinen Schema 2 erhalten:
Schema 2:
   Reaktion eines Polyglycosids mit Alkohol

      (Glyc-O)_{z}H + R¹-OH → (Glyc-O)_{z},R¹
   worin bedeuten
      - z: ≥ 1,
      - z': ≤ z, vorzugsweise 1 bis 10,
      - (Glyc-O)_{z}H: mit z = 1 Aldosen wie beispielsweise Triosen, Tetrosen, Pentosen, Hexosen, insbesondere Erythrose, Threose, Ribose, Arabinose, Xylose, Lyxose, Allose, Altrose, Glucose, Mannose, Gulose, Idose, Galactose, Fructose und Glycosidderivate, insbesondere Glucosamin, N-Acetylglucosamin, Rhamnose, Fucose, 2-Deoxy-D-erythropentose, Glucuronsäure, Galacturonsäure, Adipose, Hamamelose sowie Tetraacylglucose,
      - (Glyc-O)_{z}H: mit z > 1 Oligo- oder Polymere mit o.g. Bausteinen, gleich oder verschieden, also Oligo- oder Polyglycosiden, insbesondere Sucrose, Trehalose, Raffinose, Lactose, Cellobiose, Maltose, Isomaltulose, Lactulose, Cyclodextrin, Amylose, Cellulose, Chitin, Stärke, Inulin, Amylopectin, Pectine, Dextrane.

Besonders bevorzugte Ausgangsverbindungen der Formel (Glyc-O)_{z}H sind Glucose und ihre Verbindungen.

Als Reaktanden und gleichzeitig Lösungsmittel sind grundsätzlich alle Alkohole geeignet. In den erfindungsgemäß einsetzbaren primären oder sekundären Alkoholen der allgemeinen Formel R¹-OH bedeutet
- R¹: ein gegebenenfalls verzweigter, gegebenenfalls Doppelbindungen enthaltender Kohlenwasserstoffrest, Hydroxyalkyl-, Alkyloxy-, mit 1 bis 30 C-Atomen, insbesondere 1 bis 18, vorzugsweise 1 bis 4, wobei der Rest auch ali- oder heterocyclische Komponenten, gesättigt, ungesättigt oder aromatisch, mit einer Ringgröße von 3 bis 10 Atomen, vorzugsweise 4 bis 6 Atomen, besitzen können, welche weitere, gesättigte oder ungesättigte Kohlenwasserstoffsubstituenten mit 1 bis 30 C-Atomen, vorzugsweise 1 bis 18 C-Atome, insbesondere < C₁₀ und besonders bevorzugt < C₅ wie Methanol, Ethanol, Propanol, tragen können.

In Tabelle 1 sind beispielhaft einige Verbindungen aufgeführt.

Für das erfindungsgemäße Verfahren geeignete Verbindungen gemäß Formel II sind insbesondere Alkanole, vorzugsweise Methanol, Ethanol, Propanol, Isopropanol, Octanol, Dodecanol, Hexadecanol, Octadecanol und 2-Ethylhexanol, ferner auch Polyole, insbesondere Ethylenglycol, Diethylenglycol, Polyether, Glycerin und Trimethylolpropan. Ferner können Aminoalkohole verwendet werden, wie Ethanolamin, Diethanolamin und Triethanolamin, sowie aromatische Alkohole, insbesondere Phenol, Benzylakohol und Catechol und alicyclische Alkohole, insbesondere Cyclopentanol und Cyclohexanol, aber auch ungesättigte Alkohole, wie Hexenol, Hexadecenol und Octadecenol. Dabei ist es im Sinne der vorliegenden Erfindung auch möglich, geeignete Alkohole untereinander in Mischungen zu verwenden, wobei es ggf. ausreicht, wenn für eine der Alkoholkomponenten die überkritischen Bedingungen erreicht werden.

Die Reaktion kann gegebenenfalls in Gegenwart geeigneter homogener und heterogener Katalysatoren durchgeführt werden, ausgewählt aus der Gruppe der Protonensäuren, insbesondere HCl, H₂SO₄, H₃PO₄, Essigsäure, Zitronensäure, oder der Salze, insbesondere AlCl₃, LiClO₄, LiCl, ZnCl₂, BiCl₃, Ti(OiPr)₄ (OiPr = Isopropanolat), Seltenerd-heptafluorodimethyloctandionate (= fod) und -trifluormethansulfonate (= OTf), insbesondere Yb(fod)₃, Eu(fod)₃, Sc(OTf)₃, Yb(OTf)₃, oder der Ionenaustauscher, insbesondere Amberlyst-15, oder der Puffer, insbesondere Na₃PO₄/H₃PO₄. Die direkte Umsetzung erfolgt vorzugsweise unter Verwendung hochkonzentrierter Suspensionen monomerer Glycoside, Oligo- oder Polyglycoside in Alkoholen, vorzugsweise ohne Zusatz von Aktivatoren oder homogener Katalysatoren.

Die erfindungsgemäß verwendeten oder mitverwendeten Geräte sind geeignete Reaktoren mit Rührer zur Vorlage der Reaktionspartner, sowie eine Pumpe zur Kompression der Alkohole auf oder über den kritischen Druck. Eine geeignete Pumpe mit Suspensionskugelventilen wird beispielsweise von der Firma LEWA angeboten. Um eine Reaktion zu gewährleisten, muss sowohl die kritische Temperatur als auch der kritische Druck des Alkohols erreicht und vorzugsweise überschritten werden. Um eine ausreichende Reaktionsgeschwindigkeit zu erhalten, werden die kritischen Parameter bevorzugt um 5 bis 15 % überschritten. In Tabelle 1 sind einige Beispiele kritischer Parameter von Alkoholen aufgeführt. Die Reaktion läuft in einem beheizbaren Reaktor ab, der kontinuierlich oder diskontinuierlich betrieben werden kann.

**Tabelle 1:**

| Kritische Daten ausgewählter Reaktionsalkohole: | | |
|---|---|---|
| Alkohole | Tₖ/K | pₖ/bar |
| Methanol | 512,6 | 80,9 |
| Ethanol | 513,9 | 61,4 |
| n-Propanol | 536,8 | 51,7 |
| 2-Propanol | 508,4 | 47,6 |
| Butanol | 563,0 | 44,2 |
| Octanol | 625,5 | 28,6 |
| Hexadecanol | 770,0 | 16,1 |
| Octadecanol | 790,0 | 12,8 |
| Glycerin | 726,05 | 66,9 |

| | | |
|---|---|---|
| Tₖ = kritische Temperatur pₖ = kritischer Druck | | |

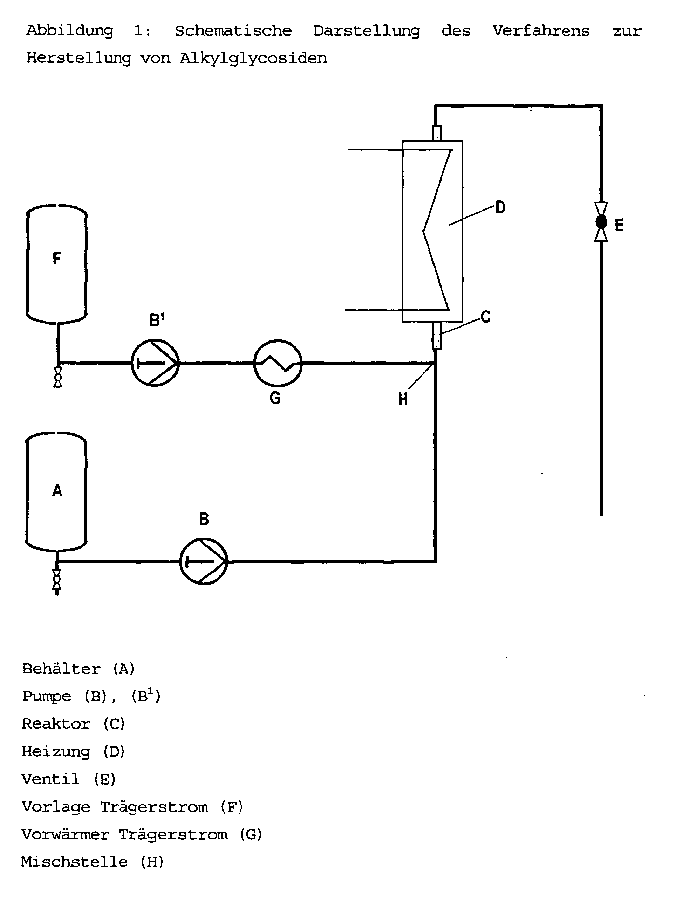

Die erfindungsgemäß verwendete Apparatur ist in Abbildung 1 schematisch dargestellt. Die Edukte werden in einem geeigneten Rühr-Behälter (A) vorgelegt. Aus dieser Vorlage wird das Gemisch durch eine geeignete Pumpe (B) in den Reaktor (C) gefördert. Die Pumpe ist dabei in der Lage das Gemisch auf einen Druck auf oder über den kritischen Druck des Alkohols gemäß Formel (II) in A zu bringen. Über die Heizung (D) wird der Reaktor (C) auf eine Temperatur größer oder gleich der kritischen Temperatur des Alkohols erwärmt. Am Ausgang des Reaktors (C) kann über ein Ventil (E) der Druck im Reaktionsaufbau reguliert werden. Ferner lassen sich so die Verweilzeiten individuell auf die jeweilige Glycosid bzw. das Glycosid/Poly-/Oligoglycosidgemisch einstellen, wodurch ein Abbau minimiert werden kann. Zur Minimierung von Nebenprodukten kann ein Trägerstrom mit reinem Alkohol aus der Vorlage (F) mittels der Pumpe B¹ über den Vorwärmer (G) auf eine Temperatur von 100 °C bis 800 °C, vorzugsweise mindestens auf die kritische Temperatur aufgeheizt werden. Am Mischpunkt (H) wird dann der Trägerstrom (TS) mit dem Eduktstrom (ES) vermischt und dem Reaktor zugeführt. Das Verhältnis von TS/ES kann in weiten Bereichen von 0/100 bis 99/1, vorzugsweise 20/80 bis 80/20, liegen. Ist die Vorwärmtemperatur so hoch gewählt, dass am Mischpunkt bereits eine Reaktionstemperatur vorliegt, ist der Temperaturgradient im Reaktor ausreichend niedrig einzustellen, um Verbrennungen zu verhindern.

Zur Identifikation der Reaktionsprodukte haben sich insbesondere GC, GC-MS, HPLC und MALDI als geeignete analytische Methoden erwiesen.

### Beispiele:

Analytik wurde in allen Beispielen sofort im Anschluss an die Experimente mit NMR und GC/MS durchgeführt. Alle Beispiele wurden in einer Apparatur nach Abbildung 1 durchgeführt. Zur Förderung der Reaktionsgemische werden Pumpen vom Typ LEWA TYP EK08 mit Pumpenkopf HK 8 mm verwendet.

### Anwendungsbeispiel 1:

### Herstellung von Methylglucosid:

Eine Mischung aus Methanol und Glucose wird in einem gerührten Gefäß A vorgelegt. Der Anteil an Glukose beträgt 30 % des Methanol-Anteils. Die Mischung wird durch eine geeignete Pumpe B bei einem Druck von 120 bar kontinuierlich durch den Reaktor C gefördert. Der Rohrreaktor C wird durch die Heizung D auf eine Temperatur von 160 °C geheizt. Ein Trägerstrom mit reinem Methanol aus der Vorlage F wird über den Vorwärmer G auf ca. 300 °aufgeheizt. Am Mischpunkt H wird der Trägerstrom mit dem Eduktstrom vermischt und dem Reaktor zugeführt. Die Vorwärmtemperatur ist so hoch gewählt, dass am Mischpunkt bereits Reaktionstemperatur vorliegt, so dass der Temperaturgradient im Reaktor ausreichend niedrig ist, um Verbrennungen an der Wand zu verhindern. Die Verweilzeit im Rohrreaktor liegt bei ca. 2 min. Unter zu Hilfenahme des Regelventils E wird der Druck auf dem genannten Sollwert gehalten. Am Ausgang des Systems wird die reagierte Reaktionsmischung aufgefangen. Laut HPLC enthält das Produktgemisch 3 % Produkt.

### Anwendungsbeispiel 2:

### Herstellung von Methylfructosid:

Eine Mischung aus Methanol und Fruktose wird in einem gerührten Gefäß A vorgelegt. Der Anteil an Fruktose beträgt 30 % des Methanol-Anteils. Die Mischung wird durch eine geeignete Pumpe B bei einem Druck von 120 bar kontinuierlich durch den Reaktor C gefördert. Der Rohrreaktor C wird durch die Heizung D auf eine Temperatur von 160 °C geheizt. Ein Trägerstrom mit reinem Methanol aus der Vorlage F wird über den Vorwärmer G auf ca. 300 °aufgeheizt. Am Mischpunkt H wird der Trägerstrom mit dem Eduktstrom vermischt und dem Reaktor zugeführt. Die Vorwärmtemperatur ist so hoch gewählt, dass am Mischpunkt bereits Reaktionstemperatur vorliegt, so dass der Temperaturgradient im Reaktor ausreichend niedrig ist, um Verbrennungen an der Wand zu verhindern. Die Verweilzeit im Rohrreaktor liegt bei ca. 2 min. Unter zu Hilfenahme des Regelventils E wird der Druck auf dem genannten Sollwert gehalten. Am Ausgang des Systems wird die reagierte Reaktionsmischung aufgefangen. Laut HPLC enthält das Produktgemisch 4 % Produkt.

### Anwendungsbeispiel 3:

### Herstellung von Ethylglucosid:

Eine Mischung aus Ethanol und Glucose wird in einem gerührten Gefäß A vorgelegt. Der Anteil an Glukose beträgt 30 % des Ethanol-Anteils. Die Mischung wird durch eine geeignete Pumpe B bei einem Druck von 120 bar kontinuierlich durch den Reaktor C gefördert. Der Rohrreaktor C wird durch die Heizung D auf eine Temperatur von 150 °C geheizt. Ein Trägerstrom mit reinem Ethanol aus der Vorlage F wird über den Vorwärmer G auf ca. 280 °C aufgeheizt. Am Mischpunkt H wird der Trägerstrom mit dem Eduktstrom vermischt und dem Reaktor zugeführt. Die Vorwärmtemperatur ist so hoch gewählt, dass am Mischpunkt bereits Reaktionstemperatur vorliegt, so dass der Temperaturgradient im Reaktor ausreichend niedrig ist, um Verbrennungen an der Wand zu verhindern. Die Verweilzeit im Rohrreaktor liegt bei ca. 2 min. Unter zu Hilfenahme des Regelventils E wird der Druck auf dem genannten Sollwert gehalten. Am Ausgang des Systems wird die reagierte Reaktionsmischung aufgefangen. Laut HPLC enthält das Produktgemisch 10 % Produkt.

## Patentansprüche

1. Verfahren zur Herstellung von Alkylpolyglycosiden aus monomeren Glycosiden, Oligo- oder Polyglycosiden und Alkoholen, nach dem allgemeinen Schema
(Glyc-O)_{z}H + R¹-OH → (Glyc-O)_{z},R¹
worin bedeuten
z ≥ 1,
z' ≤ z, vorzugsweise 1 bis 10,
(Glyc-O)- ein Glycosidrest,
R¹ ein gegebenenfalls Mehrfachbindungen und/oder Heteroatome enthaltender Kohlenwasserstoffrest, **dadurch gekennzeichnet, dass** die Reaktion unter überkritischen Bedingungen, bezogen auf den Alkohol, durchgeführt wird.

2. Verfahren gemäß Anspruch 1, **dadurch gekennzeichnet, dass** die Reaktion bei überkritischen Drücken und überkritischen Temperaturen, bevorzugt mit Parametern, die mindestens 5 % über den kritischen Parametern der Alkohole liegen, durchgeführt wird.

3. Verfahren gemäß den Ansprüchen 1 bis 2, **dadurch gekennzeichnet, dass** die Reaktion kontinuierlich oder diskontinuierlich durchgeführt wird und das Reaktionsgemisch im Reaktor eine Verweilzeit von 1 s bis 24 h, vorzugsweise von 1 s bis 1 h, insbesondere von 1 s bis 5 min, hat.

4. Kontinuierliches Verfahren gemäß den Ansprüchen 1 bis 3, **dadurch gekennzeichnet, dass** dem Eduktstrom vor dem Reaktoreingang ein Trägerstrom des Alkohols zugemischt wird, vorgeheizt auf eine Temperatur von 100 °C bis 800 °C, vorzugsweise mindestens auf die kritische Temperatur des Alkohols.

5. Verfahren gemäß den Ansprüchen 1 bis 4, **dadurch gekennzeichnet, dass**
(Glyc-O)_{z}H mit z = 1 Aldosen wie beispielsweise Triosen, Tetrosen, Pentosen, Hexosen, insbesondere Erythrose, Threose, Ribose, Arabinose, Xylose, Lyxose, Allose, Altrose, Glucose, Mannose, Gulose, Idose, Galactose, Fructose und Glycosidderivate, insbesondere Glucosamin, N-Acetylglucosamin, Rhamnose, Fucose, 2-Deoxy-D-erythropentose, Glucuronsäure, Galacturonsäure, Adipose, Hamamelose sowie Acylglucose und
(Glyc-O)_{z}H mit z > 1 gleich oder verschieden, Oligo- oder Polyglycoside wie Sucrose, Trehalose, Raffinose, Lactose, Cellobiose, Maltose, Isomaltulose, Lactulose, Cyclodextrin, Amylose, Cellulose, Chitin, Stärke, Inulin, Amylopectin, Pectine, Dextrane sind.

6. Verfahren gemäß den Ansprüchen 1 bis 5, **dadurch gekennzeichnet, dass** als Alkohol mindestens eine Verbindung der allgemeinen Formel R¹-OH verwendet wird, in der R¹ ein gegebenenfalls verzweigter, gegebenenfalls Doppelbindungen und/oder Hetroatome enthaltender Kohlenwasserstoffrest, Hydroxyalkyl-, Alkyloxyrest mit 1 bis 30 C-Atomen, insbesondere 1 bis 18, vorzugsweise 1 bis 4 ist.

7. Verfahren gemäß den Ansprüchen 1 bis 6, **dadurch gekennzeichnet, dass** als Alkohol mindestens eine Verbindung ausgesucht aus der Gruppe Methanol, Ethanol, Propanol, Isopropanol, Butanol, Hexanol, Octanol, Decanol, Dodecanol, Hexadecanol, Octadecanol, Glycol, Glycerin, Propandiol, verwendet wird.

8. Verfahren nach Anspruch 7 **dadurch gekennzeichnet, dass** die überkritischen Bedingungen mindestens für eine der Alkoholkomponenten erreicht oder überschritten werden.

9. Verfahren gemäß den Ansprüchen 1 bis 7, **dadurch gekennzeichnet, dass** die Reaktion in Gegenwart geeigneter homogener und heterogener Katalysatoren durchgeführt wird, ausgewählt aus der Gruppe der Protonensäuren, insbesondere HCl, H₂SO₄, H₃PO₄, Essigsäure, Zitronensäure, oder der Salze, insbesondere AlCl₃, LiClO₄, LiCl, ZnCl₂, BiCl₃, Ti(OiPr)₄ (OiPr = Isopropanolat), Seltenerd-heptafluorodimethyloctandionate (= fod) und -trifluormethansulfonate (= OTf), insbesondere Yb(fod)₃, Eu(fod)₃, Sc(OTf)₃, Yb(OTf)₃, oder der Ionenaustauscher, insbesondere Amberlyst-15, oder der Puffer, insbesondere Na₃PO₄/H₃PO₄.

10. Verfahren gemäß mindestens einem der Ansprüche 1 bis 4, **dadurch gekennzeichnez, dass** (Glyc-O)_{z}H Glucose und R¹-OH Methanol bedeutet.

## Claims

1. Process for preparing alkylpolyglycosides from monomeric glycosides, oligo- or polyglycosides and alcohols according to the general scheme
(Glyc-O)_{z}H + R¹-OH → (Glyc-O)_{z},R¹
where
z ≥ 1,
z' ≤ z, preferably from 1 to 10,
(Glyc-O)- is a glycoside radical,
R¹ is a hydrocarbon radical which optionally contains multiple bonds and/or heteroatoms, which comprises carrying out the reaction under supercritical conditions with regard to the alcohol.

2. Process according to claim 1, **characterized in that** the reaction is carried out at supercritical pressures and supercritical temperatures, preferably with parameters which are at least 5% above the critical parameters of the alcohols.

3. Process according to claims 1 and 2, **characterized in that** the reaction is carried out continuously or batchwise and the reaction mixture has a residence time in the reactor of 1 s to 24 h, preferably of from 1 s to 1 h, in particular of from 1 s to 5 min.

4. Continuous process according to claims 1 to 3, **characterized in that** a carrier stream of the alcohol preheated to a temperature of from 100°C to 800°C, preferably at least to the critical temperature of the alcohol, is mixed with the reactant stream upstream of the reactor inlet.

5. Process according to claims 1 to 4, **characterized in that**
(Glyc-O)_{z}H where z = 1 are aldoses, for example trioses, tetroses, pentoses, hexoses, in particular erythrose, threose, ribose, arabinose, xylose, lyxose, allose, altrose, glucose, mannose, gulose, idose, galactose, fructose and glycoside derivatives, in particular glucosamine, N-acetylglucosamine, rhamnose, fucose, 2-deoxy-D-erythropentose, glucuronic acid, galacturonic acid, adipose, hamamelose and acylglucose, and
(Glyc-O)_{z}H where z > 1 are the same or different and are oligo- or polyglycoside such as sucrose, trehalose, raffinose, lactose, cellobiose, maltose, isomaltulose, lactulose, cyclodextrin, amylose, cellulose, chitin, starch, inulin, amylopectin, pectins, dextrans.

6. Process according to claims 1 to 5, **characterized in that** the alcohol used is at least one compound of the general formula R¹-OH in which R¹ is an optionally branched hydrocarbon radical optionally containing double bonds and/or heteroatoms, or a hydroxyalkyl, alkyloxy radical having from 1 to 30 carbon atoms, in particular from 1 to 18, preferably from 1 to 4.

7. Process according to claims 1 to 6, **characterized in that** the alcohol used is at least one compound selected from the group of methanol, ethanol, propanol, isopropanol, butanol, hexanol, octanol, decanol, dodecanol, hexadecanol, octadecanol, glycol, glycerol, propanediol.

8. Process according to claim 7, **characterized in that** the supercritical conditions are attained or exceeded at least for one of the alcohol components.

9. Process according to claims 1 to 7, **characterized in that** the reaction is carried out in the presence of suitable homogeneous and heterogeneous catalysts selected from the group of the protic acids, in particular HCl, H₂SO₄, H₃PO₄, acetic acid, citric acid, or of the salts, in particular AlCl₃, LiClO₄, LiCl, ZnCl₂, BiCl₃, Ti(OiPr)₄ (OiPr = isopropoxide), rare earth heptafluorodimethyloctanedionates (= fod) and trifluoromethanesulphonates (= OTf), in particular Yb(fod)₃, Eu(fod)₃, Sc(OTf)₃, Yb(OTf)₃, or of the ion exchangers, in particular Amberlyst-15, or of the buffers, in particular Na₃PO₄/H₃PO₄.

10. Process according to at least one of claims 1 to 4, **characterized in that** (Glyc-O)_{z}H is glucose and R¹-OH is methanol.

## Revendications

1. Procédé de préparation d'alkylpolyglycosides à partir de glycosides monomères, d'oligoglycosides ou de polyglycosides et d'alcools, conformément au schéma général
(Glyc-O)_{z}H + R¹-OH → (Glyc-O)_{z},R¹
dans lequel
z ≥ 1,
z' ≤ z, valant de préférence de 1 à 10,
(Glyc-O)- représente un radical glycoside,
R¹ représente un radical hydrocarboné renfermant éventuellement des liaisons multiples et/ou des hétéroatomes, **caractérisé en ce que** la réaction est réalisée dans des conditions supercritiques, en ce qui concerne l'alcool.

2. Procédé selon la revendication 1, **caractérisé en ce** la réaction est réalisée à des pressions supercritiques et à des températures supercritiques, de préférence avec des paramètres qui sont supérieurs d'au moins 5 % aux paramètres critiques des alcools.

3. Procédé selon les revendications 1 à 2, **caractérisé en ce que** la réaction est réalisée en continu ou en discontinu et le mélange réactionnel présente un temps de séjour dans le réacteur de 1 seconde à 24 heures, de préférence de 1 seconde à 1 heure, en particulier de 1 seconde à 5 minutes.

4. Procédé continu selon les revendications 1 à 3, **caractérisé en ce qu'**un courant vecteur de l'alcool, préchauffé à une température de 100°C à 800°C, de préférence au moins à la température critique de l'alcool, est mélangé avec le courant de réactifs en amont de l'entrée du réacteur.

5. Procédé selon les revendications 1 à 4, **caractérisé en ce que**
(Glyc-O)_{z}H, avec z = 1, représente des aldoses tels que, par exemple, des trioses, des tétroses, des pentoses, des hexoses, en particulier l'érythrose, le thréose, le ribose, l'arabinose, le xylose, le lyxose, l'allose, l'altrose, le glucose, le mannose, le gulose, l'idose, le galactose, le fructose et des dérivés de glycosides, en particulier la glucosamine, la N-acétylglucosamine, le rhamnose, le fucose, le 2-désoxy-D-érythropentose, l'acide glucuronique, l'acide galacturonique, l'adipose, l'hamamélose ainsi qu'un acylglucose, et
(Glyc-O)_{z}H, avec z > 1, identique ou différent, représente des oligoglycosides ou des polyglycosides tels que le sucrose, le tréhalose, le raffinose, le lactose, le cellobiose, le maltose, l'isomaltulose, le lactulose, la cyclodextrine, l'amylose, la cellulose, la chitine, l'amidon, l'inuline, l'amylopectine, des pectines, des dextranes.

6. Procédé selon les revendications 1 à 5, **caractérisé en ce que** l'on utilise, en tant qu'alcool, au moins un composé de formule générale R¹-OH, dans laquelle R¹ représente un radical hydrocarboné éventuellement ramifié, renfermant éventuellement des doubles liaisons et/ou des hétéroatomes, un radical hydroxyalkyle ou un radical alkyloxy renfermant de 1 à 30 atomes de carbone, en particulier de 1 à 18, de préférence de 1 à 4.

7. Procédé selon les revendications 1 à 6, **caractérisé en ce que** l'on utilise, en tant qu'alcool, au moins un composé choisi parmi le groupe constitué du méthanol, de l'éthanol, du propanol, de l'isopropanol, du butanol, de l'hexanol, de l'octanol, du décanol, du dodécanol, de l'hexadécanol, de l'octadécanol, du glycol, du glycérol et du propanediol.

8. Procédé selon la revendication 7, **caractérisé en ce que** les conditions supercritiques sont atteintes ou dépasseés au moins pour l'un des composants alcools.

9. Procédé selon les revendications 1 à 7, **caractérisé en ce que** la réaction est effectuée en présence de catalyseurs homogènes et hétérogènes appropriés, choisis parmi le groupe constitué des acides protiques, en particulier du HCl, du H₂SO₄, du H₃PO₄, de l'acide acétique, de l'acide citrique, ou des sels, en particulier de l'AlCl₃, du LiClO₄, du LiCl, du ZnCl₂, du BiCl₃, du Ti(OiPr)₄ (OiPr = isopropanolate), d'heptafluorodiméthyloctanedionate (= fod) et de trifluorométhanesulfonates (= OTf) de terres rares, en particulier du Yb(fod)₃, du Eu(fod)₃, du Sc(OTf)₃, du Yb(OTf)₃, ou des échangeurs d'ions, en particulier de l'Amberlyst-15, ou des tampons, en particulier du Na₃PO₄/H₃PO₄.

10. Procédé selon au moins l'une des revendications 1 à 4, **caractérisé en ce que** (Glyc-O)_{z}H représente le glucose et R¹-OH représente le méthanol.
